# EUROPEAN PATENT SPECIFICATION

(11) **EP 1 534 168 B1**
(45) Date of publication and mention of the grant of the patent: **02.06.2010**
(21) Application number: 03723590.0
(22) Date of filing: 06.05.2003
(51) Int. Cl.: A61C 8/00, A61F 2/02, A61L 27/06

(54) **AN IMPLANT AND A METHOD FOR TREATING AN IMPLANT SURFACE**
IMPLANTAT UND VERFAHREN ZUR BEHANDLUNG EINER IMPLANTATFLÄCHE
IMPLANT ET PROCEDE DE TRAITEMENT D'UNE SURFACE D'IMPLANT

(30) Priority: 19.07.2002 SE 0202271
(43) Date of publication of application: 01.06.2005
(73) Proprietor: Astra Tech AB, 431 21 Mölndal (SE)
(72) Inventor: PETERSSON, Ingela, S-414 68 Göteborg (SE); JUNEMO-BOSTRÖM, Kristina, S-415 06 Göteborg (SE); JOHANSSON-RUDÉN, Gunilla, S-436 42 Askim (SE); ANDERSSON, Fredrik, SE-431 42 Mölndal (SE); HANSSON, Stig, S-436 39 Askim (SE); ELLINGSEN, Jan-Eirik, NO-1356 Bekkestua (NO)
(74) Representative: Andersson, Inga-Lill
(86) International application number: PCT/SE2003/000722
(87) International publication number: WO 2004/008984

(56) References cited:
- WO-A1-95/17217
- WO-A1-96/16611
- US-A- 4 330 891

## Description

### Technical field

The invention relates to an implant for implantation into bone tissue, and to a method for treating an implant surface intended for implantation into bone tissue to improve the biocompatibility of an implant comprising said surface.

### Background of the invention

A one-stage procedure is nowadays, in most cases, used for implanting orthopaedic or dental implants, generally metallic implants, into bone tissue.

In the one-stage procedure, a first implant part, such as a dental fixture, is surgically placed into the bone tissue, and a healing cap or a secondary implant part, such as an abutment, is then attached to the first implant part directly after the surgical operation. The soft tissue is thereafter allowed to heal around the healing cap or the secondary implant part. When a healing cap is used, the cap is removed after a few weeks or months without any surgical procedure, and secondary implant parts, such as an abutment and a provisional crown, are attached to the first implant part. The one-stage procedure is for instance described in L Cooper et al: "A multicenter 12-month evaluation of single-tooth implants restored 3 weeks after 1-stage surgery", The International Journal of Oral & Maxillofacial Implants, Vol 16, No 2 (2001).

The two-stage procedure, which in some dental cases still might be necessary to use, involves in a first stage surgically placing a first implant part, such as a dental fixture, into the bone tissue, where it is then allowed to rest unloaded and immobile for a healing period of three months or more in order to allow the bone tissue to grow onto the implant surface to permit the implant to be well attached to the bone tissue, the cut in the soft tissue covering the implant site being allowed to heal over the implant, and in a second stage opening the soft tissue covering the implant and attaching secondary implant parts, such as a dental abutment and/or a restoration tooth, to the first implant part, such as said fixture, forming the final implant structure. This procedure is for instance described by Brånemark et al: "Osseointegrated Implants in the Treatment of the Edentulous Jaw, Experience from a 10-year period", Almquist & Wiksell International, Stockholm, Sweden.

However, the fact that the implant should not be loaded during the healing period means that the secondary implant parts may not be attached to the first implant part and/or used during the healing period of three months or more. In view of the discomfort associated with this, it is desirable to minimize the time period necessary for the above-mentioned first stage or even perform the entire implantation procedure in a single operation, i.e. to use the one-stage procedure.

For some patients, it might be considered better to wait at least three months before functionally loading the implant, both for one- and two-stage procedures. However, an alternative using the one-stage procedure is to put the implant in function directly after implantation (immediate loading) or a few weeks after implantation (early loading). These procedures are, for instance, described by D M Esposito, pp 836-837, in Titanium in Medicine, Material Science, Surface Science, Engineering, Biological Responses and Medical Application, Springer-Verlag (2001).

It is essential that the implant establish a sufficient stability and bond between implant and bone tissue to enable the above disclosed immediate or early loading of the implant.

It shall also be noted that an immediate or early loading of the implant may be beneficial to bone formation.

Some of the metals or alloys, such as titanium, zirconium, hafnium, tantalum, niobium, or alloys thereof, that are used for bone implants are capable of forming a relatively strong bond with the bone tissue, a bond which may be as strong as the bone tissue per se, sometimes even stronger. The most notable example of this kind of metallic implant material is titanium and alloys of titanium whose properties in this respect have been known since about 1950. This bond between the metal and the bone tissue has been termed "osseointegration" by Brånemark et al.

Although the bond between the metal, e.g. titanium, and the bone tissue may be comparatively strong, it is desirable to enhance this bond.

There are to date several methods for treating metallic implants in order to obtain a better attachment of the implant, and thus improved osseointegration. Some of these involve altering the morphology of the implant, for example by creating relatively large irregularities on the implant surface in order to increase the surface roughness in comparison to an untreated surface. An increased surface roughness gives a larger contact and attachment area between the implant and the bone tissue, whereby a better mechanical retention and strength may be obtained. A surface roughness may be provided by, for example, plasma spraying, blasting or etching.

Rough etching of implant surfaces may be performed with reducing acids, such as hydrofluoric acid (HF) or mixtures of hydrochloric acid (HCl) and sulfuric acid (H₂SO₄). The aim of such a rough etching process is to obtain implant surfaces with rather large irregularities, such as pore diameters within the range of 2-10 µm and pore depths within the range of 1-5 µm.

Other methods for obtaining a better attachment of the implant to the bone tissue involve alteration of the chemical properties of the implant surface. For example, one such method involves the application of a layer of ceramic material, such as hydroxyapatite, to the implant surface, inter alia in order to stimulate the regeneration of the bone tissue. Ceramic coatings, however, may be brittle and may flake or break off from the implant surface, which may in turn lead to an ultimate failure of the implant.

Besides the above disclosed methods of implant surface modification, it shall be noted that in contact with oxygen, titanium, zirconium, hafnium, tantalum, niobium and their alloys are instantaneously covered with a thin oxide layer. The oxide layers of titanium implants mainly consist of titanium(IV) dioxide (TiO₂) with minor amounts of Ti₂O₃ and TiO. The titanium oxide generally has a thickness of about 4-8 nm. However, titanium implants having an oxide layer thickness of up to about 20 µm may be produced using anodisation (anodic oxidation). As the titanium oxide layer thickness increases, the porosity and surface roughness of the oxide layer increases. Furthermore, the crystallinity of the titanium oxide increases as the oxide layer thickness increases. Thus, an implant surface roughness may also be obtained by providing a thicker oxide layer.

Our prior application WO 95/17217 describes a process wherein a metallic implant (blasted or non-blasted) is treated with an aqueous solution of hydrofluoric acid of concentration up to 3%, most preferably about 0.2%, for a treatment period of at least 10 seconds, such as 10 seconds to 6 hours, preferably 30 s for 0.2% Hf at room temperature. According to WO 95/17217, the implant surface morphology is unaffected by this treatment, i.e. no significant etching of the surface occurs. The implant is said to have an improved biocompatibility due to retaining of fluorine and/or fluoride on the implant surfaces.

Document WO9616611 defines a titanium implant having a microroughness consisting of cone-shaped elements. The cone-shaped elements have a cone height range (approx.) = 0.30 to 0.50 micron and a cone base diameter range (approx.) = 0.30 to 0.60 micron.

Document US4330891 defines titanum implant having a microroughness of micro-pitted type (moon surface type) having pits with a diameter in the range of from about 10 nm up to about 1000 nm.

### Disclosure of the invention

An object of the present invention is to provide an implant for implantation into bone tissue having an improved rate of attachment between the implant and the bone tissue such that the post-surgery healing period described above (either using a one- or two-stage procedure) is reduced and/or an immediate or early loading of the implant is enabled.

Another object of the invention is to provide an implant forming a mechanically stronger bond with bone tissue. Thus, an implant intended for implantation into bone tissue having an improved biocompatibility is to be provided.

A method for treating an implant surface intended for implantation into bone tissue, comprises providing, on the implant surface, a microroughness comprising pores and peaks having a pore diameter of ≤ 1 µm, such as from 1 nm to 1 µm, preferably within the range of 50 nm to 1 µm, a pore depth of ≤ 500 nm, such as from 1 nm to 500 nm, preferably within the range of from 50 to 500 nm, and a peak width, at half the pore depth, of from 15 to 150% of the pore diameter.

An embodiment of the method comprises treating a metallic implant surface with an aqueous solution of hydrofluoric acid having a concentration of preferably less than 0.5 M, more preferably 0.1 M, resulting in etching, for an etching period of preferably up to 180 sec, more preferably up to 60 sec, at room temperature (24 ± 1°C). Thus, a microroughness as specified above is provided.

It has been shown that surprisingly good biocompatibility results are obtained for an implant, implanted into bone tissue, having an implant surface comprising said fine microroughness as specified above. Both an improved rate of attachment, and a stronger bond between the implant surface and the bone tissue are obtained.
Thus, the fine microroughness improves the osseointegration process.

According to the invention, said objects and other objects are achieved with an implant for implantation into bone tissue having an implant surface at least part of which, such as 0.1-99.9 area%, has been treated with the method as described herein above.

According to the invention said objects and other objects are achieved with an implant for implantation into bone tissue having an implant surface, wherein at least a part of the implant surface, such 0.1-99.9 area%, comprises a microroughness which comprises peaks and pores having a pore diameter of ≤ 1 µm, such as from 1 nm to 1 µm, preferably within the range of 50 nm to 1 µm, a pore depth of ≤ 500 nm, such as from 1 nm to 500 nm, preferably within the range of from 50 to 500 nm, and a peak width, at half the pore depth, of from 15 to 150% of the pore diameter.

Other features and advantages of the present invention will become apparent from the embodiments described hereinafter and the appended claims.

### Brief description of the drawings

Figure 1 defines the terms "pore diameter" (D), "pore depth" (h) and "peak width at half the pore depth" (x).
Figure 2 shows SEM pictures of a coarse-blasted reference implant surface.
Fig 3 shows SEM pictures of the herein described and analysed prior art implant surface according to WO 95/17217. The implant surface is non-blasted.
Fig 4 shows SEM pictures of an embodiment of the implant surface according to the present invention. The implant surface is non-blasted and has been treated according to method I (Example 1, non-blasted).
Fig 5 shows SEM pictures of an embodiment of the implant surface according to the present invention. The implant surface has been coarse-blasted and treated according to method I (Example 1, coarse-blasted).
Fig 6 shows SEM pictures of an embodiment of the implant surface according to the present invention. The implant surface is non-blasted and has been treated according to method II (Example 2, non-blasted).
Fig 7 shows SEM pictures of an embodiment of the implant surface according to the present invention. The implant surface has been coarse-blasted and treated according to method II (Example 2, coarse-blasted).
Fig 8 illustrates the AFM profile of the surface shown in Fig 3 (prior art implant).
Fig 9 illustrates the AFM profile of the surface shown in Fig 4 (Example 1, non-blasted).
Fig 10 illustrates the AFM profile of the surface shown in Fig 6 (Example 2, non-blasted).

### Detailed description of the invention

As used herein in connection with the invention the term "etching" refers to the process taking place during the treatment period during which H₂ (g) is generated at the implant surface. The etching period is measured from the formation of the first bubble of H₂(g) at the implant surface. Etching in the context of the present invention relates to a very mild etching of an implant surface providing the desired microroughness described herein.

As used herein the term "microroughness" refers to a surface roughness comprising pores having a pore diameter equal to or less than 1 µm and a pore depth equal to or less than 1 µm.

As used herein the term "macroroughness" refers to a surface roughness comprising surface irregularities having dimensions greater than 1 µm.

As used herein the term "implant" includes within its scope any device intended to be implanted into the body of a vertebrate animal, in particular a mammal, such as a human. Implants may be used to replace anatomy and/or restore any function of the body.

Generally, an implant is composed of one or several implant parts. For instance, a dental implant usually comprises a dental fixture coupled to secondary implant parts, such as an abutment and/or a restoration tooth. However, any device, such as a dental fixture, intended for implantation may alone be referred to as an implant even if other parts are to be connected thereto.

As used herein the term "implant (intended) for implantation into bone tissue" refers to implants intended for at least partial implantation into bone tissue, such as dental implants, orthopaedic implants, and the like. An implant for implantation into bone tissue may also be referred to as a bone tissue implant.

As used herein the term "implant surface" refers to at least one defined surface region of an implant. Thus, the defined surface region may include the entire surface area of the implant or portions thereof.

An example of an implant surface intended for implantation into bone tissue is the surface of a dental fixture that is intended for implantation into the jaw-bone of a patient and to be in contact with bone tissue.

Another example of an implant surface intended for implantation into bone tissue is the surface of a hip joint implant that is intended for implantation into the neck of the femur of a patient.

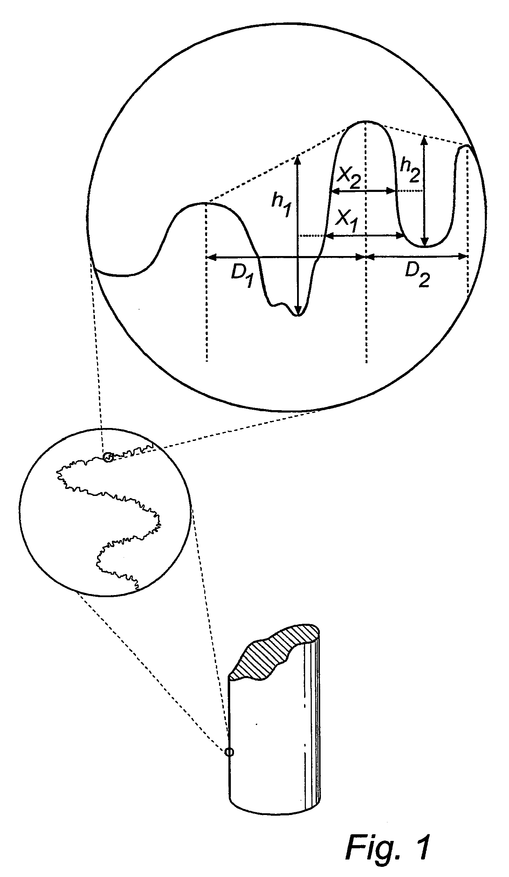

The terms "pore diameter" (D), "pore depth" (h) and "peak width at half the pore depth" (x) are defined in Fig 1. These terms are used in the context of a microroughness. In Fig 1, a microroughness is superimposed on a macroroughness. However, the same terms are used for a microroughness provided on a surface lacking said macroroughness.

The pore diameter (D) is the distance between the highest points of two adjacent peaks as defined in Fig 1. If there are several points at the same level that could be referred to as the highest, the point closest to the pore should be chosen. If the "peaks" are very broad (i.e. the surface might seem to lack well-defined peaks), the surface may be described as having an essentially flat surface profile in-between the pores (forming said microroughness), which are spread over the surface. In that case, the pore diameter is the distance between those points where the surface profile start to deviate from the essentially flat surface profile, thus forming said pore. This is in compliance with said definition shown in Fig 1.

The pore depth (h) is defined as the distance between an imaginary line drawn between the highest points of two adjacent peaks, and the bottom of the pore (at the lowest point) (see Fig 1). The distance is measured in a direction perpendicular to the tangent of said lowest point of the pore. If there are several points at the lowest level, a mean value of the distances from these points to the imaginary line is calculated as the pore depth. If no well-defined peaks are present, the imaginary line is drawn between those points where the surface profile start to deviate from an essentially flat surface profile, thus forming said pore.

The peak width (x) at half the pore depth (h) is defined as shown in Fig 1. A method for treating an implant surface intended for implantation into bone tissue, comprises providing a microroughness comprising pores and peaks having a pore diameter of ≤ 1 µm, a pore depth of ≤ 500 nm, and a peak width, at half the pore depth, of from 15 to 150% of the pore diameter.

Thus, the peak width is preferably within the range of 7.5 nm to 1.5 µm. Even more preferably are peaks having a peak width, at half the pore depth, of from 30 to 150% of the pore diameter. Most preferably are peaks having a peak width, at half the pore depth, of from 60 to 150% of the pore diameter

This specific surface morphology gives a very resistant bone in-growth. With this specific morphology, newly formed bone, which grows into the surface irregularities of the implant surface, does not easily fracture from the old bone. In addition, the peaks of the implant surface do not easily fracture from the implant.

Furthermore, it shall be noted that only a part or parts of the implant surface may comprise the herein specified surface irregularities, which means that the pores and peaks may be grouped in several regions throughout the surface. Thus, the distances between pores and/or peaks may vary throughout the surface. Preferably, > 10 area% of the implant surface comprises said surface irregularities, more preferably > 40 area%, and still more preferably ≥ 70 area%. Most preferably, the entire implant surface comprises said surface irregularities homogeneously distributed throughout the surface.

A surface roughness is often evaluated using atomic force microscopy (AFM). From such a measurement a root-mean-square roughness (R_{q} and/or S_{q}) may be obtained. The root-mean-square roughness corresponds to the root-mean-square deviation of the profile from the mean line over one sampling length. Rq is the root-mean-square roughness measured in two dimensions and S_{q} is the root-mean-square roughness measured in three dimensions.

AFM is a very sensitive method of surface characterisation. However, the analysis must be carefully executed so that the microroughness is analysed and not the underlying surface structure, such as a blasted or machined surface.

The root-mean-square roughness may also be calculated based upon the surface morphology shown on SEM pictures of the implant surface or estimated from results obtained by any other method of surface characterisation.

Thus, calculations using a pore diameter of ≤ 1 µm and a pore depth of ≤ 500 nm gives a root-mean-square roughness (R_{q}) of ≤ 250 nm based upon the definition of root-mean-square roughness (R_{q}) as know to persons skilled in the art.

The implant surface is preferably a metallic implant surface, such as a titanium implant surface.

The metallic implant surface might be part of a metallic implant or it might be an applied metallic surface layer of a non-metallic implant, such as a ceramic, a plastic or a composite material. Furthermore, the metallic implant surface might also be part of a partly metallic implant, whereby a partly metallic implant surface is provided.

A microroughness as specified according to the invention may be provided using mild etching, micro fabrication, anodisation, flame spraying, electrochemical treatment, laser, spark erosion, or any other suitable method of surface modification.

Preferably, the microroughness is provided by treating the metallic implant surface with an aqueous solution of hydrofluoric acid (HF), resulting in an etching process.

The concentration of the hydrofluoric acid is preferably less than 0.5 M, more preferably 0.1 M.

The metallic implant surface is preferably treated for an etching period of up to 180 sec, more preferably up to 60 sec, at room temperature (24 ± 1°C). Before the etching starts the natural oxide layer is removed by the acid and when the acid gets in contact with the implant surface, the etching process starts and the above disclosed microroughness is provided by the etching process of the implant surface.

The treatment with HF(aq) is preferably performed at room temperature, i.e. at about 20-30°C (normal air pressure), preferably 24 ± 1°C. If a higher temperature than 24 ± 1°C is used, the etching process will, as known to a person skilled in the art, be initiated earlier and the etching process will be more rapid, i.e. a shorter etching period than the period given herein for etching at 24 ± 1°C is needed to obtain the desired result. Hence, if a lower temperature than 24 ± 1°C is used, a longer etching period than the period given herein for etching at 24 ± 1°C is needed to obtain the desired result.

The etching period, the temperature and the concentration of HF (aq) determines the ratio between etched areas, i.e. areas having a microroughness, and non-etched areas.

Preferably, the method further comprises providing a macroroughness on the implant surface prior to providing the microroughness. Thus, an implant having a microroughness superimposed on the macroroughness is obtained. The macroroughness is preferably provided by blasting, more preferably blasting a titanium implant surface with titanium dioxide particles.

A macroroughness may also be provided by any other suitable technique, such as etching, micro fabrication, anodisation, flame spraying, any electrochemical treatment, laser, spark erosion, machining, knurling, or any other suitable method of surface modification.

Furthermore, it shall also be noted that the implant surface, with or without a macroroughness, may be either unthreaded or threaded.

Said metallic implant surface is preferably made of commercially pure titanium or an alloy of titanium, but it may also be made of any other biocompatible metallic material, such as zirconium or an alloy thereof, hafnium or an alloy thereof, niobium or an alloy thereof, tantalum or an alloy thereof, a chromium-vanadium alloy, or any combination of these materials.

The implant for implantation into bone tissue according to the invention is preferably a dental implant or an orthopaedic implant.

The present invention relates to an implant for implantation into bone tissue having an implant surface at least part of which has been treated with the method according to the invention as described herein above.

Thus, an implant for implantation into bone tissue having an implant surface with the above described characteristics forms the present invention.

The invention will now be illustrated by means of the following non-limiting examples.

### EXAMPLES

### Sample preparation

Surgical implants of commercially pure (c.p.) titanium were used.

Each implant was ultrasonically degreased in Ecosolv^{®} (70-100% ethyl-2-hydroxypropionate) for 5 min, and thereafter in ethanol (70%) for 5 min.

Some of the implants were thereafter blasted with titanium dioxide particles. Two different particle size ranges of titanium dioxide were used; 6.8-90 µm (fine = F), and 106-180 µm (coarse = C). However, coarser particles sizes, such as 180-300 µm, may also be used.

The blasted implants were then ultrasonically rinsed in deionised water for 2 x 5 min, and in ethanol for 2 x 5 min to remove any residual blasting particles.

The implants were then treated according to the following methods:

### a) Reference implants

Non-blasted and blasted (F and C) implants, cleaned in accordance with above, were provided as references for the studies as described hereinafter.

### b) Prior art method (according to WO 95/17217)

Non-blasted and blasted implants (F and C), cleaned in accordance with above, were immersed in 0.1 M HF (aq) at room temperature (about 24 ± 1°C) for 90 s. No H₂ (g) was formed during this treatment period, thus no etching occurred.

The implants were thereafter immersed in deionised water for 20 s, and thereafter dried.

### c) Method I

Non-blasted and blasted implants (F and C), cleaned in accordance with above were immersed in ethanol (99.5% for 2 s and in deionised water for 5 s.

The implants were thereafter, according to the present invention, immersed in a stirred solution of 0.1 M HF (aq) at room temperature (about 24 ± 1°C) for an etching period of 40 ± 5 sec. About 80-90 area% of the surface was then etched, thus providing a microroughness. However, since the etching process was shown to be slower for non-blasted implants, these implants should preferably be etched for a longer time period, such as 60 ± 5 sec, than blasted implants to obtain a similar degree of etching. The etching period was measured from the formation of the first bubble of H₂ (g) at the implant surface. The etching of the implant surface starts when the acid is in direct contact with the pure titanium, i.e. when the titanium oxide covering the titanium surface is removed.

The implants were thereafter immersed in stirred deionised water for 20 s.

The implants were ultrasonically rinsed in ethanol (20%) for 3 min, and in deionised water for 4 min.

The implants were then rinsed in ethanol (99.5%) for 5 s, wiped, and dried.

An implant treated in accordance with this method is referred to as Example 1.

### d) Method II

Non-blasted and blasted (F and C) implants, cleaned in accordance with above, were immersed in ethanol (99.5%) for 2 s and in deionised water for 5 s.

The implants were thereafter, according to the present invention, immersed in 0.1 M HF (aq) at room temperature (about 24 ± 1°C) with stirring for an etching period of 40 ± 5 sec. Due to reasons explained above, some of the non-blasted implants were etched for 60 ± 5 sec (these samples were only used for the AFM measurement described hereinafter). The etching period was measured from the formation of the first bubble of H₂ (g) at the implant surface.

The implants were then wiped and dried.

An implant treated in accordance with this method is referred to as Example 2.

### In vivo evaluation

Implant surfaces treated in accordance with the above methods were evaluated in vivo using the tensile test described in Biomaterials 23 (2002), pp 2201-2209, by H J, Ronald, and J E Ellingsen.

The implants were in the form of coins having a diameter of 6.25 mm and a height of 1.95 mm. One side of the implant coins were treated with said methods. In the centre of the other side of the coin, a threaded hole for attachment to a load cell was provided.

New Zeeland white rabbits were used as test animals. Two guide holes were drilled in one of each rabbit's tibial bone using a 1.0 mm diameter twist drill (Medicon^{®}, Germany) using a drill guide to ensure a standardised and correct positioning. Cavities were then prepared for each implant coin using a custom made 7.05 mm diameter stainless steel bur mounted in a slow speed dental implant drill with copious physiological saline solution irrigation.

The treated and untreated implant surfaces, according to Table 1, were placed in the cavities and stabilised by a pre-shaped 0.2 mm titanium maxillofacial plate (Medicon^{®} CMS, Germany), retained in the cortical bone by two 1.2 x 3 mm² titanium screws (Medicon^{®} CMS, Germany). This ensured a stable passive fixation of the implants during the healing period. Polytetrafluorethylene (PTFE) caps were introduced to resist bone growth towards the vertical faces of the implant as well as bone overgrowth. The subcutaneous soft tissue and the superficial layers were repositioned and sutured.

The treated surface was in direct contact with the bone tissue, but the vertical sides and the reverse side of the coin were not in contact with bone tissue.

The implant coins were then left for 7 weeks in test 1, and for 8 weeks in test 2.

18 rabbits were used in test 1, and 20 rabbits were used in test 2.

At the end of said period, the rabbits were sacrificed, and the implant fixations and the PTFE caps were removed. The tibial bone was then fixed in a specially designed rig to stabilise the bone during the test procedure. A threaded pin with a ball-head was attached to the implant coin by use of the pre-made threaded hole and the set-up was adjusted perpendicularly to the load cell using a level tube. Tensile tests were then performed using a Lloyds LRX Materials testing machine fitted with a calibrated load cell of 100 N. Cross-head speed range was set to 1.0 mm/min. Load was applied until the implant detached from the bone and the force applied was recorded on a load versus displacement plot. The detachment of the implant coin was in this plot indicated as a well-defined breakpoint with a vertical drop in load. The mean values of the forces needed to pull out the differently treated coins are given in Table 1. The recorded force gives a direct assessment of the strength of connection between the implant coin and the bone. The higher the recorded force, the stronger the connection.

The first test included a reference coin blasted with fine (F) titanium dioxide particles, and blasted (F) coins treated in accordance with, method I, and method II as outlined above.

The second test included a reference coin blasted with fine (F) titanium dioxide particles, a reference coin blasted with coarse (C) titanium oxide particles, and blasted (C) coins treated in accordance with method I and method II as outlined above.

**Table 1**

| | Reference implant | | Example 1 | | Example 2 | |
|---|---|---|---|---|---|---|
| Blasting particles | F | C | F | C | F | C |
| Test 1: Recorded force [N] | 18.3 | - | 29.0 | - | 26.2 | - |
| Test 2: Recorded force [N] | 17.1 | 32.2 | - | 39.8 | - | 38.2 |

As can be seen from Table 1, the implant coins treated in accordance with method I and II gave an improved bone attachment as compared to the reference coins.

Furthermore, it shall be noted that the coin implants blasted with coarse (C) titanium oxide particles gave a better bone attachment than coin implants blasted with fine (F) titanium oxide particles.

### Surface characterisation

The surface characteristics of implants treated.in accordance with the methods disclosed above were evaluated using Atomic Force Microscopy (AFM), and Scanning Electron Microscopy (SEM).

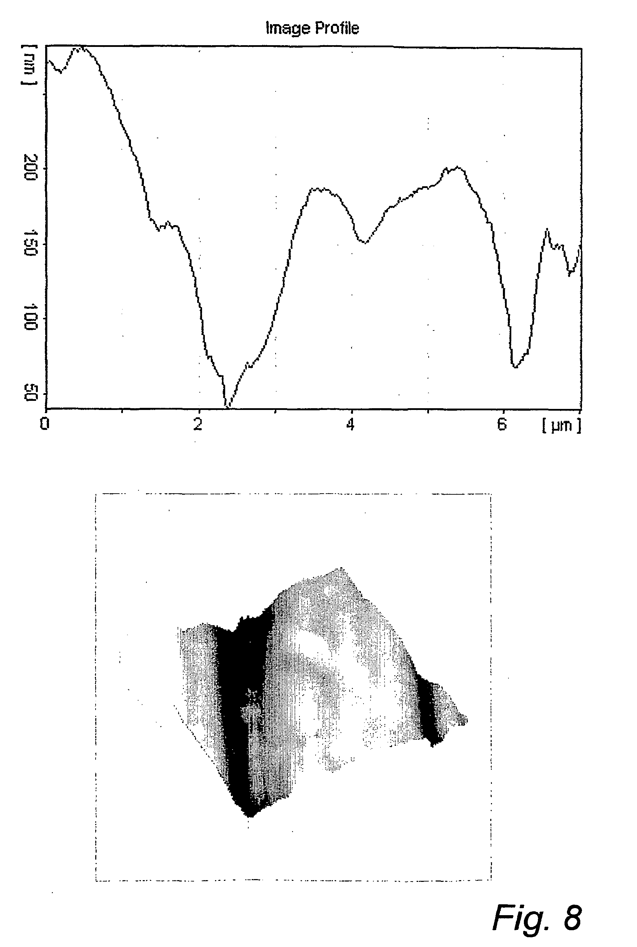

AFM (AFM DualScope, DME AS, Denmark) was used to measure the morphology of the implant surfaces. Two sizes of sample areas were measured, 5 x 5 µm (256 points sampling in x- and y-direction) and 10 x 10 µm (256 points sampling in x- and y-direction), respectively (see Fig 8-10). The z-scaling of the 3D-pictures (5 x 5 µm) shown in Fig 8-10 has been increased four times.

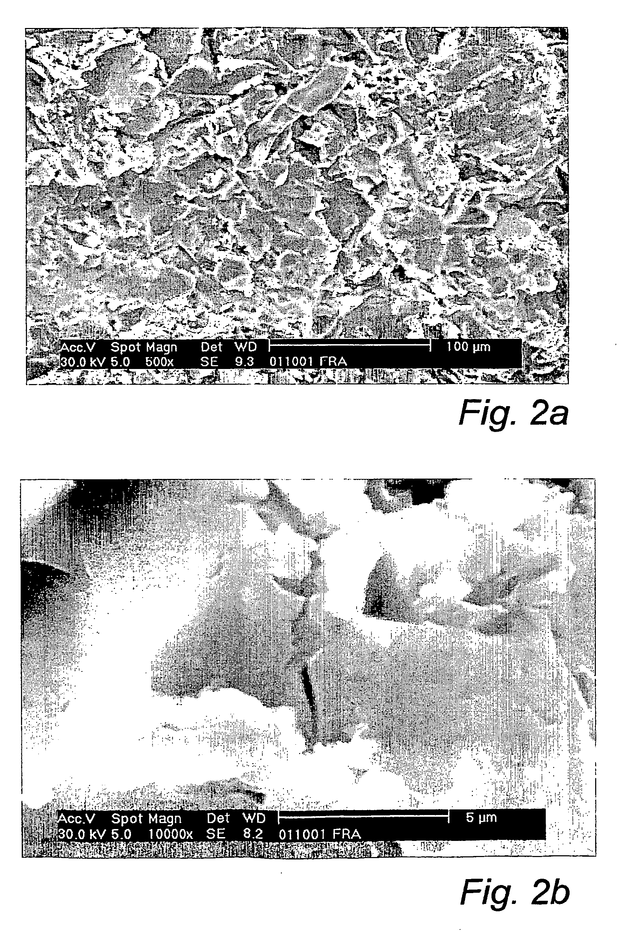

SEM (Philips XL-30 ESEM, Philips, the Netherlands) was used to visually study the surface morphology (see Fig 2-7).

The surface characteristics for implants treated in accordance with the methods disclosed above were evaluated. Non-blasted implants and implants blasted with coarse (C) titanium dioxide particles were studied.

The implant surfaces were studied by SEM and AFM.

SEM pictures of an untreated, coarse-blasted (C) reference implant surface are shown in Fig 2 (magnification x500, and x10 000).

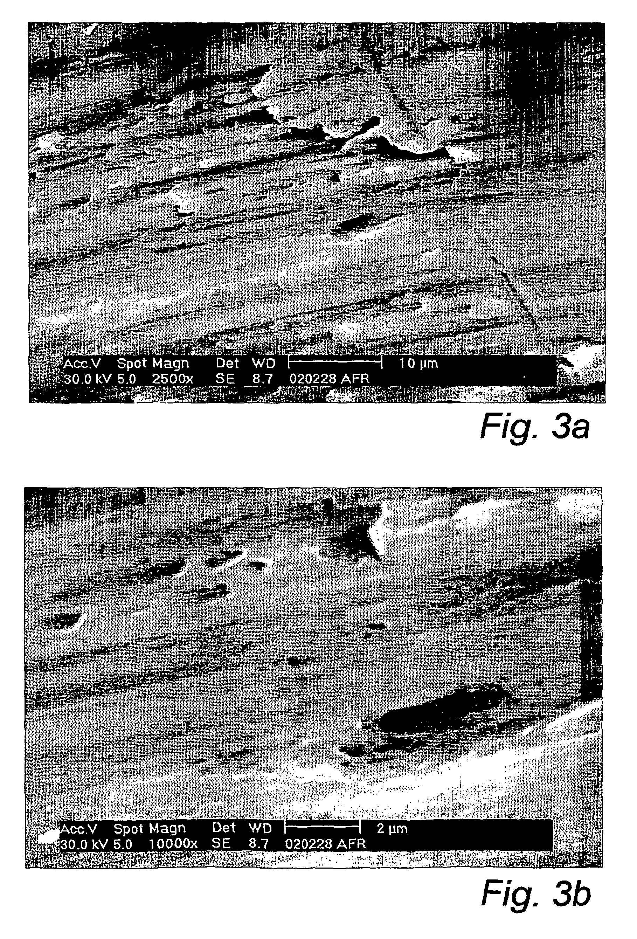

SEM pictures of the non-blasted implant surface treated according to the prior art method described above are shown in Fig 3 (magnification x2 500, and x10 000). An AFM graph of this surface is shown in Fig 8.

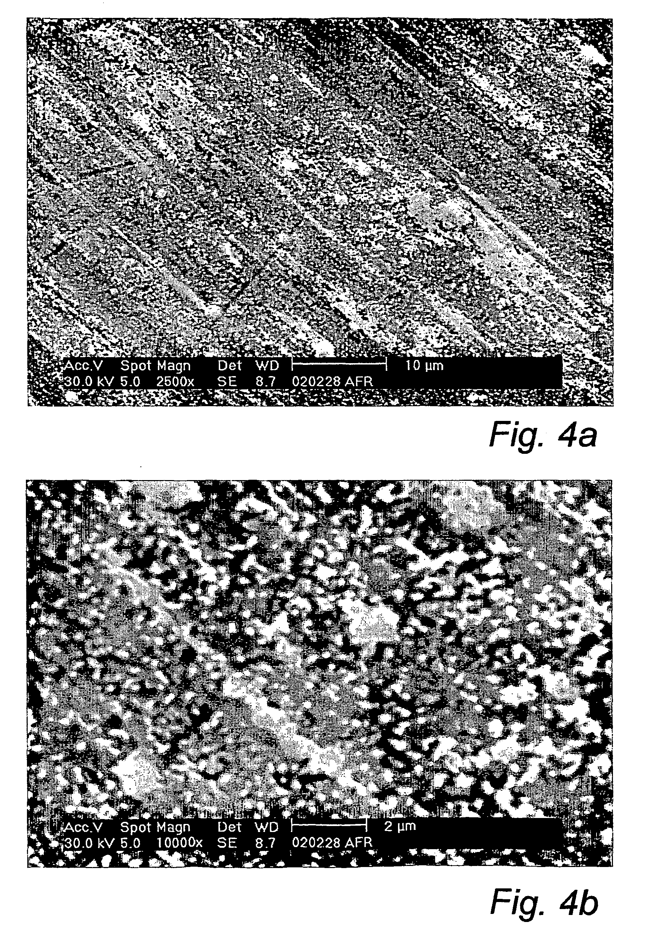

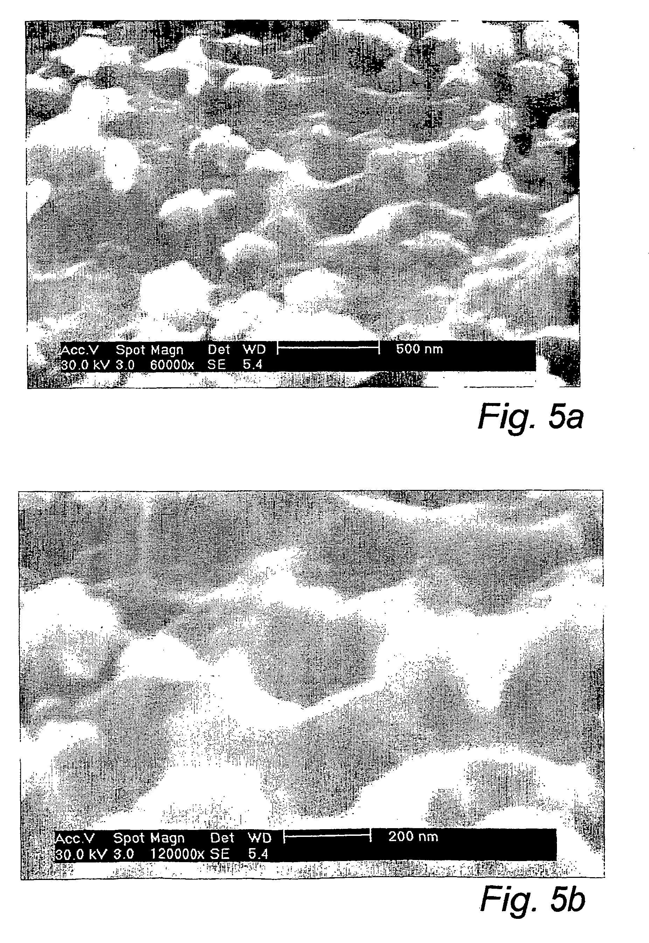

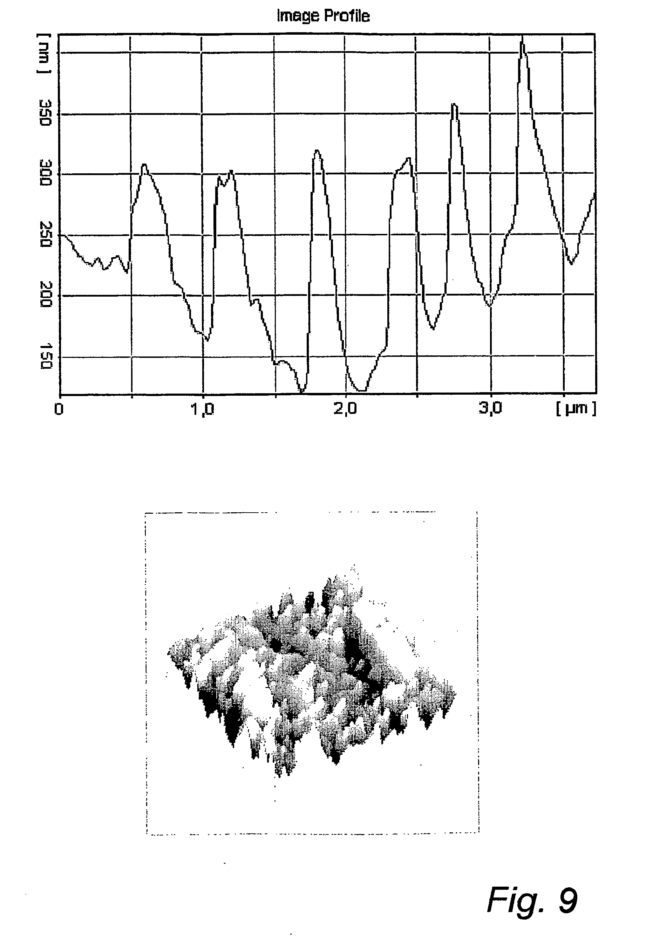

SEM pictures of the non-blasted and coarse-blasted (C) implant surfaces treated according to method I are shown in Fig 4 (magnification x2 500, and x10 000) and Fig 5 (magnification x60 000 and x120 000), respectively. An AFM graph of the non-blasted surface shown in Fig 4 is shown in Fig 9.

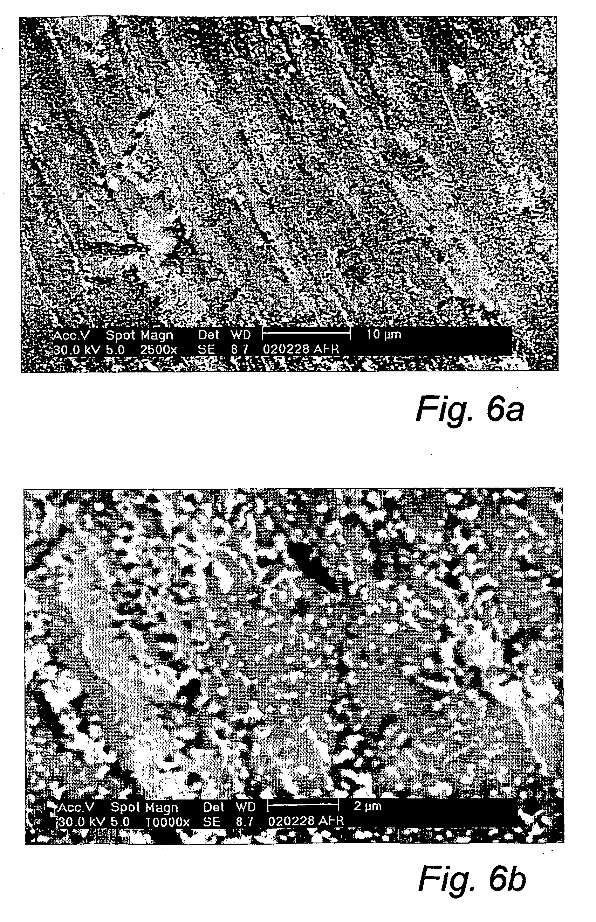

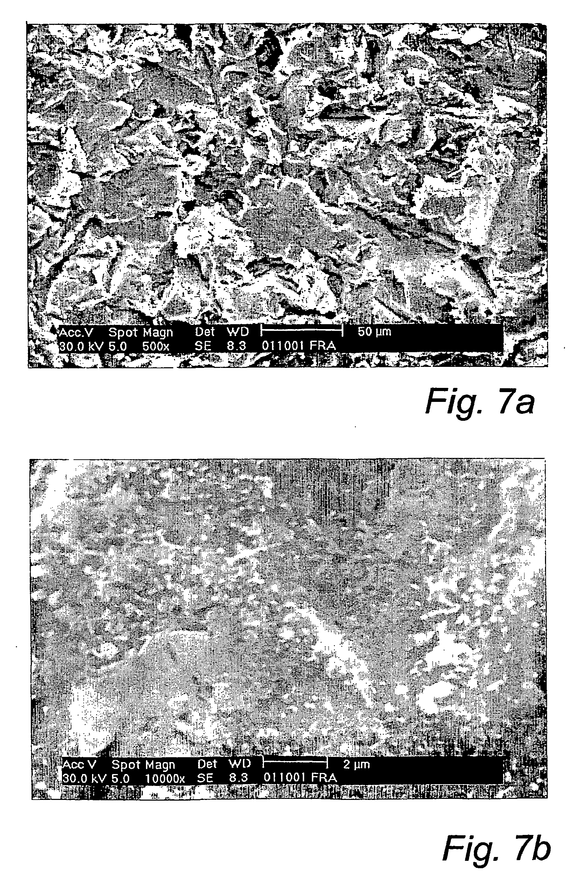

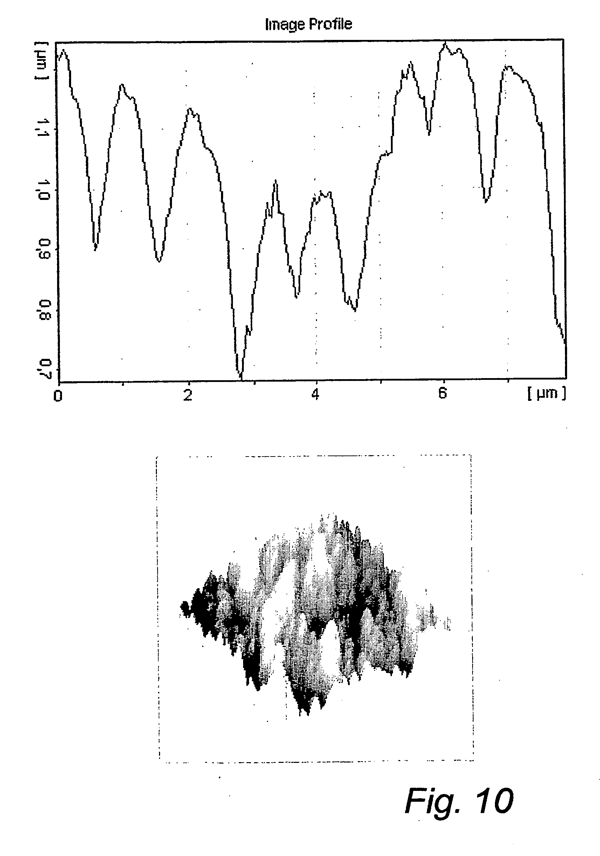

SEM pictures of the non-blasted and coarse-blasted (C) implant surfaces treated according to method II are shown in Fig 6 (magnification x2 500, and x10 000) and Fig 7 (magnification x500, and x10 000), respectively. An AFM graph of the non-blasted surface shown in Fig 6 is shown in Fig 10.

The results indicated that both blasted and non-blasted implants treated according to method I and II had pores with a pore diameter of 100-600 nm, more specifically predominantly around 250-300 nm, a pore depth of 50-300 nm, more specifically predominantly around 60-150 nm, and a peak width, at half the pore depth, of 150-670 nm.

The microroughness parameters obtained for the non-blasted surfaces using AFM are given in Table 2. Parameter values for two regions of the implant surface were recorded and these values are given in Table 2

**Table 2**

| | Reference implant | Prior art implant | Example 1 | Example 2 |
|---|---|---|---|---|
| Blasting particles | no blast | no blast | no blast* | no blast** |
| Measured area: 10 x 10 µm | | | | |
| Sₐ [µm] | 0.04 | 0.06 | 0.13 | 0.12 |
| | 0.04 | 0.05 | 0.08 | 0.10 |
| S_{q} [µm] | 0.04 | 0.07 | 0.16 | 0.14 |
| | 0.05 | 0.07 | 0.10 | 0.12 |
| S_{dr} [%] | 1.1 | 1.9 | 49.3 | 20.0 |
| | 2.0 | 1.8 | 40.3 | 10.7 |

| Measured area: 5 x 5 µm | | | | |
|---|---|---|---|---|
| Sₐ [µm] | 0.03 | 0.02 | 0.10 | 0.09 |
| | 0.04 | 0.04 | 0.07 | 0.09 |
| S_{q} [µm] | 0.03 | 0.03 | 0.12 | 0.11 |
| | 0.04 | 0.05 | 0.08 | 0.11 |
| S_{dr} [%] | 1.5 | 1.2 | 46.8 | 19.7 |
| | 2.4 | 5.3 | 35.8 | 12.2 |

| | | | | |
|---|---|---|---|---|
| * Etching period: 40 ± 5 sec ** Etching period: 60 ± 5 sec | | | | |

As can be seen in Table 2, the Sₐ and S_{q} are about 0.07-0.13 µm and 0.08-0.16 µm, respectively, for the implants of Example 1 and Example 2, which are embodiments of the present invention.

Furthermore, the surface developed ratio (S_{dr}) , i.e. the increase of surface area as compared to a smooth area, is increased for the implants of Example 1 and Example 2 in comparison to the reference and prior art implant.

Moreover, it can be seen from Table 2 that the surface morphology of the implant surface treated according to the prior art method is similar to the reference implant surface, i.e. the surface is unaffected, which is also confirmed by the SEM pictures (Fig 3). The values obtained (shown in Table 2) are most likely due to machine traces.

To improve the accuracy and to obtain higher resolution of the AFM measurement for blasted surfaces, the AFM scanner was placed in an vibration damping sample stage. A blasted (C) surface treated according to method I was analysed with this modified instrument set-up. These values are given in Table 6.

**Table 3**

| | Example 1 |
|---|---|
| Blasting particles | C |
| Measured area: 5 x 5 µm | |
| Sₐ [µm] | 0.19 |
| | 0.11 |
| S_{q} [µm] | 0.22 |
| | 0.13 |
| S_{dr} [%] | 26.89 |
| | 50.89 |

As can be seen in Table 3, the Sₐ and S_{q} are about 0.11-0.19 µm and 0.13-0.22 µm, respectively, for the coarse-blasted implant of Example 1.

The SEM pictures (see Fig 4-7) and the AFM results (see Fig 9 and Fig 10) show that the microroughness of blasted and non-blasted surfaces treated according to the method of the present invention, i.e. in this example method I and method II, are almost identical. Furthermore, it can be seen that the implant treated with the prior art method is unaffected, i.e. the surface is almost identical to the untreated reference implant.

While the invention has been described in detail and with reference to specific embodiments thereof, it will be apparent for one skilled in the art that various changes and modifications can be made therein without departing from scope of the appended claims.

## Claims

1. An implant for implantation into bone tissue having an implant surface,
wherein the implant surface is a metallic implant surface **characterized in that** at least part of the metallic implant surface comprises a microroughness which comprise pores and peaks having a pore diameter of ≤ 1 µm, a pore depth of ≤ 500 nm, and a peak width, at half the pore depth, of from 15 to 150% of the pore diameter.

2. An implant according to claim 1, wherein the pore diameter is within the range of 50 nm to 1 µm and the pore depth is within the range of 50 to 500 nm.

3. An implant according to claim 1 or claim 2, wherein the microroughness has a root-mean-square roughness (R_{q} and/or S_{q}) of ≤ 250 nm.

4. An implant according to any one of claims 1-3, wherein the implant further comprises a macroroughness.

5. An implant according to any one of the claims 1-4, wherein said implant is a metallic implant.

6. An implant according to any one of the claims 1-5, wherein said metallic implant surface is made of commercially pure titanium or an alloy of titanium.

7. An implant according to any one of claims 1-6, wherein the implant is a dental implant.

8. An implant according to any one of claims 1-6, wherein the implant is an orthopaedic implant.

## Patentansprüche

1. Implantat zur Implantation in Knochengewebe mit einer Implantatfläche, wobei die Implantatfläche eine metallische Implantatfläche ist, **dadurch gekennzeichnet, dass** mindestens ein Teil der metallischen Implantatfläche eine Mikrorauheit umfasst, welche Poren und Spitzen mit einem Porendurchmesser von ≤ 1 µm, einer Porentiefe von ≤ 500 nm und einer Spitzenbreite, bei halber Porentiefe, von 15 bis 150% des Porendurchmessers umfasst.

2. Implantat nach Anspruch 1, wobei der Porendurchmesser im Bereich von 50 nm bis 1 µm liegt und die Porentiefe im Bereich von 50 bis 500 nm liegt.

3. Implantat nach Anspruch 1 oder 2, wobei die Mikrorauheit eine quadratische Rauheit (R_{q} und/oder S_{q}) von ≤ 250 nm aufweist.

4. Implantat nach einem der Ansprüche 1-3, wobei das Implantat ferner eine Makrorauheit umfasst.

5. Implantat nach einem der Ansprüche 1-4, wobei das Implantat ein metallisches Implantat ist.

6. Implantat nach einem der Ansprüche 1-5, wobei die metallische Implantatfläche aus Titan technischer Reinheit oder einer Titanlegierung hergestellt ist.

7. Implantat nach einem der Ansprüche 1-6, wobei das Implantat ein Dentalimplantat ist.

8. Implantat nach einem der Ansprüche 1-6, wobei das Implantat ein orthopädisches Implantat ist.

## Revendications

1. Implant destiné à être implanté dans le tissu osseux ayant une surface d'implant, la surface de l'implant étant une surface d'implant métallique **caractérisé en ce qu'**au moins une partie de la surface d'implant métallique comprend une microrugosité qui comprend des pores et des pics ayant un diamètre de pores ≤ 1 µm, une profondeur de pores ≤ 500 nm, et une largeur de pics, à 50 % de la profondeur de pores, de 15 à 150 % du diamètre de pores.

2. Implant selon la revendication 1, dans lequel le diamètre de pores se trouve dans la plage de 50 nm à 1 µm et la profondeur de pores se trouve dans la plage de 50 à 500 nm.

3. Implant selon la revendication 1 ou la revendication 2, dans lequel la microrugosité a une rugosité quadratique moyenne (R_{q} et/ou S_{q}) ≤ 250 nm.

4. Implant selon l'une quelconque des revendications 1 à 3, dans lequel l'implant comprend en outre une macrorugosité.

5. Implant selon l'une quelconque des revendications 1 à 4, dans lequel ledit implant est un implant métallique.

6. Implant selon l'une quelconque des revendications 1 à 5, dans lequel ladite surface d'implant métallique est formée par du titane commercialement pur ou un alliage de titane.

7. Implant selon l'une quelconque des revendications 1 à 6, dans lequel l'implant est un implant dentaire.

8. Implant selon l'une quelconque des revendications 1 à 6, dans lequel l'implant est un implant orthopédique.
